(19) Europäisches Patentamt
European Patent Office
Office européen des brevets

(11) Publication number: 0 309 161 A2

# (12) EUROPEAN PATENT APPLICATION

(21) Application number: **88308582.1**

(22) Date of filing: **16.09.88**

(51) Int. Cl.[4]: **C07K 9/00 , A61K 37/02 , //(C12N1/02,C12R1:01)**

The applicant has filed a statement in accordance with Rule 28 (4) EPC (issue of a sample only to an expert). Accession number(s) of the deposit(s): NCIB-12531.

Claims for the following Contracting States: ES + GR.

The title of the invention has been amended (Guidelines for Examination in the EPO, A-III, 7.3).

(30) Priority: **19.09.87 GB 8722090**
**09.12.87 GB 8728809**
**15.02.88 GB 8803353**

(43) Date of publication of application:
**29.03.89 Bulletin 89/13**

(84) Designated Contracting States:
**ES GR**

(71) Applicant: **BEECHAM GROUP PLC**
**Beecham House Great West Road**
**Brentford Middlesex TW8 9BD(GB)**

(72) Inventor: **Athalye, Medha**
**Beecham Pharmaceuticals Brockham Park**
**Betchworth Surrey, RH3 7AJ(GB)**
Inventor: **Elson, Anna**
**Beecham Pharmaceuticals Brockham Park**
**Betchworth Surrey, RH3 7AJ(GB)**
Inventor: **Gilpin, Martin Leonard**
**Beecham Pharmaceuticals Brockham Park**
**Betchworth Surrey, RH3 7AJ(GB)**
Inventor: **Jeffries, Leonard Richard**
**VA 194, Vallsala**
**Javea Alicante(ES)**

(74) Representative: **West, Vivien et al**
**Beecham Pharmaceuticals Great Burgh Yew**
**Tree Bottom Road**
**Epsom Surrey KT18 5XQ(GB)**

## (54) Antibiotic compounds.

(57) Glycopeptide antibiotics MM 45289 and MM 47756 are produced by Amycolatopsis orientalis strain NCIB 12531.

EP 0 309 161 A2

# NOVEL COMPOUNDS

The present invention relates to novel antibacterially active materials obtainable from a microorganism, to processes for their production, and to their pharmaceutical use.

A large number of microorganisms have been isolated from nature and certain of those microorganisms have been found to produce various metabolites, which can be isolated and some of which have useful antibacterial activity.

EP-A-0 231 111 and EP-A-0 265 071 (USSN 909791) describe glycopeptide antibiotics which have been obtained by fermentation of microorganisms.

A first aspect of the present invention provides a compound of formula (I):

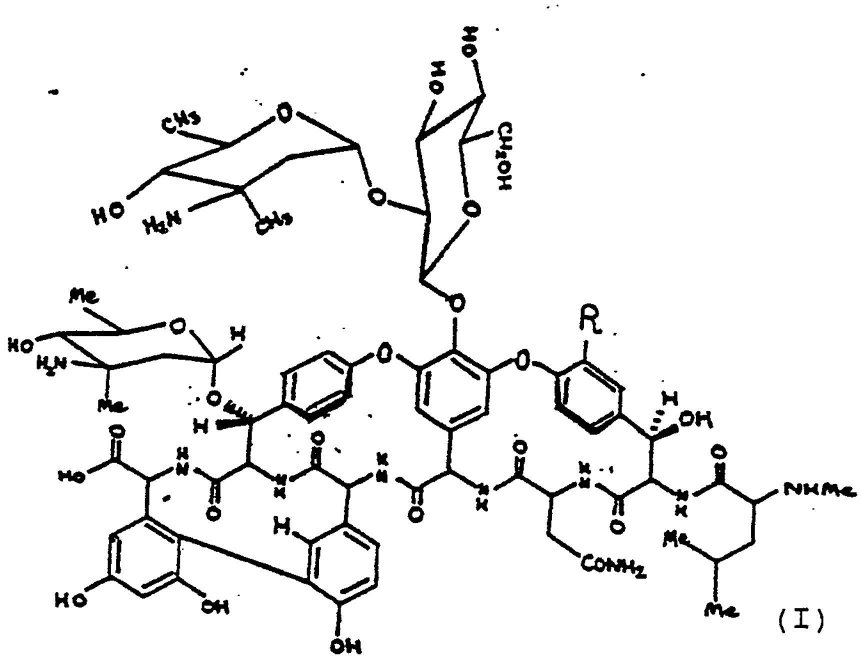

(I)

wherein R is hydrogen or chlorine, or a pharmaceutically acceptable derivative thereof, more particularly in substantially pure form.

A second aspect of the invention provides a mixture of compounds of the invention, particularly in substantially pure form.

The present invention also provides a process for the production of a compound of formula (I) which comprises cultivating a producing microorganism and subsequently isolating the compound of formula (I) or a derivative thereof from the culture.

The present invention furthermore provides a process for the preparation of a compound of formula (I) which comprises separating the compound of formula (I) or a derivative thereof from a solution thereof in admixture with other antibacterially active substances and/or inactive substances by adsorption onto an affinity resin.

The compounds of formula (I) have the characteristics set out hereinbelow in the Examples.

Each compound of formula (I) may be obtained by the cultivation of a producing microorganism and the recovery of the compound of formula (I) or a derivative thereof from the culture.

The term 'cultivation' (and derivatives of that term) as used herein means the deliberate aerobic growth of an organism in the presence of assimilable sources of carbon, nitrogen, sulphur and mineral salts. Such aerobic growth may take place in a solid or semi-solid nutritive medium, or in a liquid medium in which the nutrients are dissolved or suspended. The cultivation may take place on an aerobic surface or by submerged culture. The nutritive medium may be composed of complex nutrients or may be chemically defined.

It has been found that suitable microorganisms for use in the cultivation process according to the invention include bacterial strains belonging to the order actinomycetales that are capable of elaborating

compounds of formula (I). It has further been found that an example of such a strain is actinomycete sp. NCIB 12531 and also mutants thereof, which has been isolated from nature.

The term 'mutant' as used herein includes any mutant strain which arises spontaneously or through the effect of an external agent whether that agent is applied deliberately or otherwise. Suitable methods of producing mutant strains including those outlined by H.I. Adler in 'Techniques for the Development of Microorganisms' in 'Radiation and Radioisotopes for Industrial Microorganisms', Proceedings of a Symposium, Vienna, 1973, page 241, International Atomic Energy Authority, and these include:

(i) Ionizing radiation (e.g. X-rays and λ-rays), u.v. light, u.v. light plus a photosensitizing agents (e.g. 8-methoxypsoralen), nitrous acid, hydroxylamine, pyrimidine base analogues (e.g. 5-bromouracil), acridines, alkylating agents (e.g. mustard gas, ethyl-methane sulphonate), hydrogen peroxide, phenols, formaldehyde, heat, and

(ii) Genetic techniques, including, for example, recombination, transformation, transduction, lysogenisation, lysogenic conversion, protoplast fusion and selective techniques for spontaneous mutants.

Actinomycete sp. NCIB 12531 is believed to be a previously unreported strain in the order actinomycetales and therefore also forms a part of the present invention, particularly in biologically pure form. It has been deposited at the National Collection of Industrial and Marine Bacteria Ltd. (N.C.I.B), Torry Research Station, PO Box 31, 135 Abbey Road, Aberdeen, United Kingdom AB9 8DG under number 12531 on 7th September, 1987.

## Classification of NCIB 12531

### Methods used

The methods followed were those recommended by the International *Streptomyces* Project (ISP) for the characterization of *Streptomyces* species [Shirling, E.B. and Gottlieb, D. 'Methods for characterisation of *Streptomyces* species' Int. J. Syst. Bacteriol.,16:313-340 (1966)] and those recommended for the characterization of *Amycolata* and *Amycolatopsis* species [Lechevalier, M.P., Prauser, H., Labeda, D.A. and Ruan, J.-S. 'Two new genera of nocardioform actinomycetes: *Amycolata* cen. nov. and *Amycolatopsis* gen. nov.' Int. J. Syst. Bacteriol., 36:29-37 (1986)].

The isomers of diaminopimelic acids (DAP) and the carbohydrates in hydrolysates of whole cells were established by Thin Layer Chromatography (TLC) using the methods described by Komagata and Suzuki [Komagata, K. and Suzuki, K.-I. 'Lipid and cell-wall analysis in bacterial systematics' in Current Methods for the Classification and Identification of Microorganisms. R.R. Colwell and R. Grigorva, Eds. Methods in Microbiology vol.19. (1987) p161-207. Academic Press Inc. London, New York]. Mycolic acids were determined by the method described by Minnikin et al. [Minnikin, D.E., Hutchinson, I.G. and Caldicott, A.B. 'Thin-Layer Chromatography of Methanolysates of Mycolic Acid Containing Bacteria'. J. Chromatography 188:221-233 (1980)].

### Cultural Characteristics

Culture NCIB 12531 grew well on all the growth media recommended by the ISP, forming well developed, cream/pale yellow/pale brown substrate mycelium and abundant white aerial mycelium. No soluble pigments were detected on any of the media. The cultural characteristics of NCIB 12531 on various media are summarised in Table 1.

### Chemical Characteristics

Hydrolysed whole-cells of the culture NCIB 12531 contained the *meso* isomer of diaminopimelic acid and arabinose and galactose as the major sugars. Minor amounts of the sugars ribose and glucose were also detected in the hydrolysates. Mycolic acids were not present. These results indicate that culture NCIB 12531 has a cell-wall type IV with type A sugar pattern *sensu* Lechevalier *et al*. However, lack of mycolic acids places culture NCIB 12531 firmly outside the genus *Nocardia sensu stricto*.

Physiological Characteristics

Key physiological characteristics of cultures NCIB 12531 and Amycolatopsis orientalis ATCC 19795, listed in Table 2, show that these two cultures share many common features.

Identification of Culture NCIB 12531

Based on key chemical, physiological and morphological features, culture NCIB 12531 is identified as a strain of Amycolatopsis orientalis. Since culture NCIB 12531 does differ in some instances from the type strain of Amycolatopsis orientalis (e.g. decomposition of adenine, production of amylase and utilization of arabinose and xylose as sole carbon sources), the culture can be regarded as a new isolate of the species Amycolatopsis orientalis.

Table 1

NCIB 12531: Growth Characteristics after 14 days at 28°C

| MEDIUM | GROWTH | AERIAL MYCELIUM | | SUBSTRATE | SOLUBLE |
|---|---|---|---|---|---|
| | | FORMATION | COLOUR | | |
| ISP No.2 | Good | Good | White | Cream/pale yellow | None |
| ISP No.3 | Good | Good | White | Cream/white | None |
| ISP No.4 | Good | Good | White | Cream/pale yellow | None |
| ISP No.5 | Good | Good | White | Cream/pale brown | None |
| ISP No.6 | Good | None | - | Cream/pale brown | None |
| ISP No.7 | Good | Good | White | Cream/white | None |
| Nutrient Agar | Good | Good | White | Pale yellow | None |
| Bennetts Agar | Good | Good | White | Cream/pale brown | None |
| Czapek-Dox Agar | Good | Good | White | Cream/white | None |

Table 2

| NCIB12531: Physiological characteristics (after 14 days at 28° C) | | |
|---|---|---|
| CHARACTERISTICS | NCIB 12531 | Amycolatopsis orientalis ATCC 19795 |
| Decomposition of: | | |
| Adenine | + | - |
| Casein | + | + |
| Hypoxanthine | + | + |
| Tyrosine | + | + |
| Xanthine | + | + |
| Production of: | | |
| Amylase | + | - |
| Urease | + | + |
| Gelatinase | + | + |
| Melanin | - | - |
| Growth in the presence of: | | |
| Lysozyme (500u./ml) | + | + |
| Rifampicin (50µg/ml) | + | + |
| NaCl (5%, w/v) | + | + |
| Growth at: | | |
| 10° C | + | + |
| 28° C | + | + |
| 37° C | - | - |
| 45° C | - | - |
| Utilization of carbohydrates as sole carbon sources: | | |
| Arabinose | - | + |
| Fructose | + | + |
| Glucose | + | + |
| Inositol | - | v |
| Mannitol | + | + |
| Raffinase | - | - |
| Rhamnose | v | v |
| Sucrose | + | v |
| Xylase | v | + |
| Control (No sugar) | - | - |
| Key: V = variable | | |

The fermentation medium for cultivating sp. NCIB 12531 suitably contains sources of assimilable carbon and assimilable nitrogen together with inorganic salts. Suitable sources of nitrogen include yeast extract, soyabean flour, meat extract, cottonseed, flour, malt, distillers dried solubles, amino acids, protein hydrolysates and ammonium and nitrate nitrogen. Suitable carbon sources include glucose, lactose, maltose, starch and glycerol. Suitably the culture medium also includes alkali metal ions (for example, sodium), halogen ions (for example, chloride), and alkaline earth metal ions (for example calcium and magnesium), as well as trace elements such as iron and cobalt. The fermentation may be carried out continuously or batch-wise.

The cultivation may suitably be effected at a ure of about 20 to 35 °C . advantageously 25 to 32 °C. and the culture may suitably be harvested up to 7 days. advantageously about 3 to 5 days. after the initiation of fermentation in order to give an optimum yield of the compounds of formula (I).

The desired compound of formula (I) or a derivative thereof may then be isolated from the culture medium and worked up and purified using conventional techniques for glycopeptide compounds. All such isolation and purification procedures may conveniently be effected at cool to ambient temperature, for example at a temperature within the range of from 4 to 30 °C. conveniently from 20 to 25 °C.

The desired product can be obtained from the culture filtrate, and/or from the cell mass. Conveniently, however, the first isolation step may involve removal of solid material from the fermentation broth by, for example, filtration or centrifugation, to give a clarified culture filtrate.

Further isolation of the desired compound of formula (I) from the clarified culture filtrate may conveniently be effected by adsorption onto an affinity resin such as D-alanyl-D-alanine-Sepharose affinity resin. ("Sepharose" is a Trade Mark.)

The desired compound may readily be identified in a routine manner by testing for antibacterial activity and/or by monitoring the h.p.l.c. retention time.

Suitably, the separation procedure may include a high-performance liquid chromatography step, preferably as the last step. Elution may be effected using aqueous $NaH_2PO_4$/acetonitrile.

The compounds of formula (I) and their derivatives may be crystalline or non-crystalline and, if crystalline, may optionally be hydrated or solvated.

The compounds according to the invention are suitably provided in substantially pure form, for example at least 50% pure, suitably at least 60% pure, advantageously at least 75% pure, preferably at least 85% pure, more preferably at least 95% pure, especially at least 98% pure, all percentages being calculated as weight/weight. An impure or less pure form of a compound according to the invention may, for example, be used in the preparation of a more pure form of the same compound or of a related compound (for example a corresponding derivative) suitable for pharmaceutical use.

The compounds of formula (I) and their pharmaceutically acceptable derivatives have antibacterial properties and are useful for the treatment of bacterial infections in animals, especially mammals, including humans, in particular humans and domesticated animals (including farm animals). The compounds may be used for the treatment of infections caused by a wide range of organisms including, for example, those mentioned herein.

The present invention further provides a pharmaceutical composition comprising a compound of formula (I) or a pharmaceutically acceptable derivative thereof together with a pharmaceutically acceptable carrier or excipient.

The present invention also provides a method of treating bacterial infections in animals, especially in humans and in domesticated mammals, which comprises administering a compound of formula (I) or a pharmaceutically acceptable derivative thereof, or a composition according to the invention, to a patient in need thereof.

The compounds of the invention also have potential as growth promotors in animals. Accordingly, a further aspect of the invention provides a method of promoting weight gain and/or increasing feed utilization efficiency of livestock, which method comprises the administration of an effective non-toxic amount of a compound of the invention.

The compounds and compositions according to the invention may be formulated for administration in any convenient way for use in human or veterinary medicine, by analogy with other antibiotics.

The compounds and compositions according to the invention may be formulated for administration by any route, for example oral, topical or parenteral. The compositions may, for example, be made up in the form of tablets, capsules, powders, granules, lozenges, creams, syrups, or liquid preparations, for example solutions or suspensions, which may be formulated for oral use or in sterile form for parenteral administration by injection or infusion.

Tablets and capsules for oral administration may be in unit dosage form, and may contain conventional excipients including, for example, binding agents, for example, syrup, acacia, gelatin, sorbitol, tragacanth, or polyvinylpyrrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; and pharmaceutically acceptable wetting agents, for example sodium lauryl sulphate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or another suitable vehicle before use. Such liquid preparations may contain conventional additives, including, for example, suspending agents, for example sorbitol, methyl cellulose, glucose syrup, gelatin, hydroxyethyl

cellulose, carboxymethyl cellulose, aluminium stearate gel or hydrogenated edible fats; emulsifying agents, for example lecithin, sorbitan monooleate or acacia; non-aqueous vehicles (which may include edible oils), for example almond oil, oily esters (for example glycerine), propylene glycol, or ethyl alcohol; preservatives, for example methyl or propyl p-hydroxybenzoate or sorbic acid; and, if desired, conventional flavouring and colour agents.

Compositions according to the invention intended for topical administration may, for example, be in the form of ointments, creams, lotions, eye ointments, eye drops, ear drops, impregnated dressings, and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

Compositions according to the invention may be formulated as suppositories, which may contain conventional suppository bases, for example cocoa-butter or other glycerides.

Compositions according to the invention intended for parenteral administration may conveniently be in fluid unit dosage forms, which may be prepared utilizing the compound and a sterile vehicle, water being preferred. The compound, depending on the vehicle and concentration used, may be either suspended or dissolved in the vehicle. In preparing solutions, the compound may be dissolved in water for injection and filter-sterilised before being filled into a suitable vial or ampoule, which is then sealed. Advantageously, conventional additives including, for example, local anaesthetics, preservatives, and buffering agents can be dissolved in the vehicle. In order to enhance the stability of the solution, the composition may be frozen after being filled into the vial, and the water removed under vacuum; the resulting dry lyophilized powder may then be sealed in the vial and a accompanying vial of water for injection may be supplied to reconstitute the liquid prior to use. Parenteral suspensions may be prepared in substantially the same manner except that the compound is suspended in the vehicle instead of being dissolved and sterilisation cannot be accomplished by filtration. The compound may instead be sterilised by exposure to ethylene oxide before being suspended in the sterile vehicle. Advantageously, a surfactant or wetting agent is included in such suspensions in order to facilitate uniform distribution of the compound.

A compound or composition according to the invention may suitable be administered to the patient in an antibacterially effective amount.

A composition according to the invention may suitably contain from 0.1% by weight, preferably from 10 to 60% by weight, of a compound according to the invention (based on the total weight of the composition), depending on the method of administration.

The compounds according to the invention may suitably be administered to the patient at a daily dosage of from 1.0 to 50 mg/kg of body weight. For an adult human (of approximately 70 kg body weight), from 50 to 3000 mg, for example about 1500 mg, of a compound according to the invention may be administered daily. Suitably, the dosage for adult humans is from 5 to 20 mg/kg per day. Higher or lower dosages may, however, be used in accordance with normal clinical practice.

When the compositions according to the invention are presented in unit dosage form, each unit dose may suitably comprise from 25 to 1000 mg, preferable from 50 to 500 mg, of a compound according to the invention.

The following Examples illustrate the present invention. In the Examples, MM 45289 designates the compound of formula (I) wherein R is chlorine, and MM 47756 designates the compound of formula (I) wherein R is hydrogen.

## Example 1

### a) Fermentation.

Culture NCIB 12531 was grown for 7 days at 26° C on a solid agar slant in a McCartney bottle. The agar medium had the following composition:

| Constituent | Amount (g/l) |
|---|---|
| Yeast extract | 4.0 |
| Malt extract | 10.0 |
| Dextrose | 4.0 |
| Agar | 20.0 |
| Deionised water to 1 litre | |

[The constituents were all 'Bacto' products (Bacto is a Trade Mark) as supplied by Difco Laboratories, P.O. Box 14B, Central Avenue, East Molesey, Surrey]. The medium was adjusted to pH7.3 before sterilisation.

A spore suspension was prepared by adding 10ml of sterilised water containing 0.005% Triton X 100 to a McCartney bottle agar culture of NCIB 12531, followed by sonication of the suspension for 1 minute. 1ml of this suspension was added to a flask of seed stage medium [Triton X 100 was obtained from B.D.H Chemicals Ltd., Poole, Dorset].

The seed stage fermentation was carried out in a 500ml conical flask containing 100ml of seed stage medium. The seed stage medium contained:

| Constituent | Amount (g/l) |
|---|---|
| Soya bean flour | 10 |
| Glycerol | 20 |
| Maltose | 2 |
| Stock trace elements | 10ml |
| Deionised water to 1 litre | |

The stock trace element solution contained:

| Constituent | Amount (g/l) |
|---|---|
| $CaCl_2.2H_2O$ | 10 |
| $MgCl_2.6H_2O$ | 10 |
| NaCl | 10 |
| $FeCl_3$ | 3 |
| $ZnCl_2$ | 0.5 |
| $CuCl_2.2H_2O$ | 0.5 |
| $MnSO_4.4H_2O$ | 0.5 |
| $CoCl_2.6H_2O$ | 0.5 |

The medium was adjusted to pH7.3 before sterilisation at 117° C for 15 minutes. [The soya bean flour was Arkasoy 50 supplied by the British Arkady Co. Ltd., Old Trafford, Manchester]. Incubation was carried out for 72 hours at 26° C and 240r.p.m. on a gyratory shaker. 5ml of the seed culture was used to inoculate each of twenty 500ml conical flasks containing 100ml of fermentation medium. The fermentation medium was identical to the seed stage medium. The fermentation was carried out for 96 hours at 26° C and 240r.p.m. on a gyratory shaker. The harvested broth was clarified by centrifugation.

b) Isolation of MM 45289

The glycopeptide, MM 45289 was isolated from the clarified broth by adsorption onto D-alanyl-D-alanine-Sepharose affinity resin.

The affinity adsorbent was prepared from D-alanyl-D-alanine immobilised on Activated CH-Sepharose 4B as described in the Reference Example.

[6-Aminohexanoic acid-activated-sepharose-4B was obtained from Sigma Chemical Co., Poole. Dorset].

The clarified broth (1800ml) prepared as described in a) was stirred for 1 hour with D-alanyl-D-alanine-Sepharose affinity resin (30ml wet volume). The mixture was filtered onto a glass scinter funnel and the filtrate discarded. The affinity resin was washed with distilled water (100ml) and filtered as before. The resin was washed twice more with distilled water. The glycopeptide was eluted from the affinity resin by stirring with 0.1M ammonia containing 50% acetonitrile (50ml) for 15min. The mixture was filtered and the filtrate evaporated under reduced pressure to dryness, to yield a white solid (50mg).

The solid was re-suspended in water (50ml) and the glycopeptide adsorbed once more onto D-alanyl-D-alanine-Sepharose affinity resin (30ml wet volume). The resin was washed with water and the glycopeptide eluted with 0.1M ammonia containing 50% acetonitrile as previously described. The eluant was evaporated to dryness to yield 30mg of substantially pure MM 45289.

Fast Atom Bombardment Mass Spectroscopy indicated a molecular weight of 1556±2 for MM 45289.

The antibacterial activity of MM 45289 against a range of Gram positive organisms is shown in the following Table:

Antibacterial activity of MM 45289 against a range of organisms, determined by the microtitre method

| Organism | MM 45289 |
|---|---|
| Bacillus subtilis ATCC 6633 | 0.10 |
| Coynebacterium xerosis NCTC 9755 | 0.05 |
| Sarcina lutea NCTC 8340 | 0.05 |
| Staphylococcus aureus 'Oxford' | 0.25 |
| 'Russell' | 0.25 |
| 'V573' MR* | 1.00 |
| S.saprophyticus 'FL1' | 2.00 |
| S.epidermidis 60137 | 0.50 |
| 54815 | 0.50 |
| Streptococcus pyrogenes CN10 | <0.03 |
| S.agalactiae 'Hester' | 0.10 |
| S.sanguis ATCC 10556 | 0.50 |
| S.faecalis 1 | 1.00 |

*Multi-resistant (Methicillin, tetracycline, erythromycin and gentamycin resistant)

## Example 2

a) Fermentation.

In order to provide a large volume of inoculum, for larger scale fermentations, 400ml of seed stage medium, prepared as described in Example 1, was used to inoculate 15L of second stage seed medium in a 20L stainless steel fermenter. The medium had the same composition as the first stage medium except

for the addition of 0.1% v v polypropylene glycol P2000 antifoam. The second-stage culture was incubated at 28°C. stirred at 200rpm with a three-vaned disc impeller and sparged with sterile air at a rate of 0.5 volumes per volume per minute for 72 hours.

8L of this culture was used to inoculate a 450L stainless steel fermenter containing 300L of fermentation medium having the same composition as the second-stage seed medium. The culture was incubated at 28°C, and sparged with sterile air at a rate of 0.5 volumes per volume per minute. The culture was stirred with a three-vaned disc impeller at 50rpm, which was increased to 75rpm at 24 hours and to 100 rpm at 48 hours. Agitation was maintained at 100 rpm for the remainder of the fermentation. The fermentation was harvested at 75 hours and the broth clarified by centrifugation.

b) Isolation of MM 45289

Clarified broth from example 2a) was applied to a 12L Amberlite IRA 458 anion-exchange column ($Cl^-$ form) at a flow rate of 1.5L min. [Resin supplied by Rohm and Haas Ltd., Lenning House, 2 Mason's Avenue, Croydon, Surrey.] The percolate, containing MM 45289 was collected, adjusted to pH 6.4 and loaded onto a 15L Amberlite IRC 50 cation-exchange column ($Na^+$ form) at a flow rate of 1L min. (Resin supplied by Rohm and Haas Ltd.). The column was washed with 10 litres of water and the water wash discarded. The active material was eluted from the column with 0.2M ammonia solution at 1L min. 10L fractions were collected. Fractions with antibiotic activity (6 - 11) were bulked and evaporated in vacuo to 3.9L. $NaH_2PO_4$ was added to give a molarity of 0.005M and the pH was adjusted to 6.5 with 5M NaOH.

The concentrated IRC 50 eluate was applied to a 0.77L CM sephadex C25 cation exchange column ($Na^+$ form), previously equilibrated with 0.005M $NaH_2PO_4$ pH 6.5. [CM Sephadex was supplied by Pharmacia Ltd., Uppsala, Sweden.] The percolate was discarded, and the column was washed with 0.005M $NaH_2PO_4$ (1L) pH 7.0 and then 0.05M $NaH_2PO_4$ (1L) pH 7.0. The washings were discarded. The MM 45289 was eluted using an exponential gradient of 0.05M $NaH_2PO_4$ pH 7.0 to 0.2M $Na_2HPO_4$ pH 10.0 (1 litre in mixing vessel) at a flow rate of 20ml/min. 16ml fractions were collected. Fractions containing the glycopeptide (85 - 175) were bulked, diluted to 3L with deionised water, and the pH adjusted to 6.7 with 5M HCl. The bulked fractions were desalted using D-alanyl-D-alanine Sepharose affinity resin (100ml wet volume of resin). The resin was stirred with the bulked fractions for 1 hour and the resin recovered by filtration. The resin was washed with water (three 250ml aliquots) and eluted with four 400ml aliquots of 0.1M ammonia containing 50% acetonitrile. The percolate, which still contained some antibiotic activity was treated once more with 100ml wet volume D-alanyl-D-alanine Sepharose affinity resin and the resin recovered, washed and eluted as described above. The eluates and water wash fractions containing MM 45289 were combined and the ammonia and acetonitrile evaporated off in vacuo. $NaH_2PO_4$ was added to the concentrated solution (1900ml) to give a molarity of 0.005M, and the pH was adjusted to 6.6 with 5M NaOH.

The buffered solution was applied to a 320ml CM Sephadex C25 column previously equilibrated in 0.005M $NaH_2PO_4$ pH 6.6, at a flow rate of 10ml min. The column was eluted using an exponential gradient of 0.05M $NaH_2PO_4$ pH 7.0 to 0.2M $Na_2HPO_4$ pH 10.0 (500ml in mixing vessel). 16ml fractions were collected. Those fractions containing MM 45289, (65 - 140) were combined and adjusted to pH 6.5 with 5M HCl.

The solution was stirred for 1 hour with D-alanyl-D-alanine Sepharose affinity resin (90ml wet volume). The resin was recovered by filtration, washed with four 400ml aliquots of deionised water and the MM 45289 eluted with 0.1M ammonia containing 50% acetonitrile (three 200ml aliquots). The percolate and water washes, which still contained some antibiotic activity were combined and retreated with D-alanyl-D-alanine Sepharose affinity resin as described above. This procedure was repeated twice more. The eluate fractions were all combined and evaporated in vacuo to remove the ammonia and acetonitrile. The solution was finally freeze-dried to yield 3.33g of substantially pure MM 45289. The solid was dried over $P_2O_5$ prior to analysis.

Example 3

a) Fermentation

A spore suspension (1ml) of NCIB 12531 in 10% glycerol, 5% lactose, 0.01% Tween 80 in deionised water, which had been stored in liquid nitrogen, was used to inoculate one 500ml flask containing 100ml of the following medium:

| Constituent | Amount (g/l) |
|---|---|
| Glucose | 15.0 |
| Dextrin | 20.0 |
| Soya bean flour (Arkasoy 50) | 15.0 |
| Corn Steep Liquor (spray dried) | 10.0 |
| Yeast extract | 1.0 |
| $CaCO_3$ | 2.0 |
| Deionised water to 1 litre. | |
| pH adjusted to 6.7 before sterilisation at 121 °C for 15 minutes. | |

[Corn Steep Liquor was supplied by Roquette (UK) Ltd, Tunbridge Wells, Kent, UK. Other constituents were from suppliers named in Example 1.]

The flask was incubated at 26 °C for 72 hours at 240rpm on a gyratory shaker.

5ml portions of the seed stage were used to inoculate a further eight 500ml flasks containing 100ml of the same medium. These flasks were incubated under the same conditions but for 48 hours.

400ml of the second seed stage was used to inoculate 15L of the same medium but containing polypropylene glycol P2000 antifoam (0.1% v/v), and made up in tap water, in a 20L fermenter. The medium was sterilised, prior to inoculation, at 121 °C for 60 minutes. The fermentation was stirred with an agitator fitted with 3 vaned-discs at 200rpm, sterile air was supplied at 0.5 volumes per volume per minute and incubation was at 28 °C for 48hrs.

8 litres of the third stage seed medium was used to inoculate 300L of production medium in a 450L fermenter. The medium was identical to the medium described in Example 1, but containing polypropylene glycol P2000 antifoam (0.1% v/v). The fermenter was stirred with an agitator fitted with 3 vaned-discs at 50rpm, sterile air was supplied at 0.5 volumes per volume per minute. The culture was incubated at 28 °C for 76 hours.

The culture broth (300L) was harvested at 76 hours.

b) Isolation of MM 45289

The clarified broth was applied to a 12.5L Amberlite IRA 458 anion-exchange column ($Cl^-$ form) at a flow rate of 1L/min. The column was washed with 15L of water. The percolate and water wash were combined and the pH adjusted to 6.8

A 16.5L IRC 50 cation exchange column (Na+ form) was equilibrated by washing with 50L of 0.05M $NaH_2PO_4$ pH 6.5, followed by 50L of deionised water. The combined IRA458 percolate and water wash was loaded onto the column at a flow rate of 1L/min. The column was washed with 20L of deionised water and the percolate and washing discarded.

The active material was eluted from the column with 0.2M ammonia solution at 1L/min. 10 litre fractions were collected. Fractions with antibiotic activity (9 - 16) were bulked, adjusted to pH 7.0 with acetic acid and evaporated to 4.2L.

The concentrated IRC eluate was loaded onto a 0.79L CM Sephadex C25 cation-exchange column ($Na^+$ form), previously equilibrated with 0.005M $NaH_2PO_4$ pH 6.5. The column was washed with 0.05M $NaH_2PO_4$ pH 7.0 (1L) and the percolate and washing discarded. The antibiotic activity was eluted using an exponential gradient of 0.05M $NaH_2PO_4$ pH 7.0 to 0.2M $Na_2HPO_4$ pH 10.2 (1 litre in mixing vessel). 19ml fractions were collected. Fractions containing MM 45289 (65 - 145) were bulked (1,540ml) and adjusted to pH 7.0).

The bulked CM Sephadex fractions were diluted to 5 litres and desalted on a 1.5L Diaion HP2O column

[supplied by Mitsubishi Chemical Industries. Tokyo, Japan]. The flow rate was 80ml min. The column was eluted with deionised water, three 500ml fractions were collected and then 250ml fractions. Fractions containing MM 45289 (6 - 11) were bulked and freeze-dried to yield 10.3g of substantially pure MM 45289. The solid was dried over $P_2O_5$ prior to analysis.

Example 4

Preparation of MM 45289 by preparative HPLC

MM 45289, prepared as described in Examples 1 to 3. although substantially free of impurities, may contain 3 - 4% of MM 47756. The MM 47756 may be removed by column chromatography using CM Sephadex cation-exchange resin as described in Example 6 for the preparation of MM 47756. Residual traces of MM 47756 may be removed from samples of MM 45289 by preparative hplc as described below.

100mg of substantially pure MM 45289, but containing 3% MM 47756 was dissolved in 1ml of 0.1M sodium phosphate buffer pH 6.0. 2 x 250μl aliquots were applied to a Dynamax 150-A preparative hplc column, 10mm x 300mm [Ramin Instruments Co., Woburn, MA, USA] containing C18 reverse phase material. The column was eluted isocratically with 0.1M sodium phosphate buffer pH 6.0 containing 5% acetonitrile, at a flow rate of 6.0ml/min. The eluant was monitored at 210nm on a Waters Lambda-Max model 481 spectrophotometer, using a semi-preparative flow cell.

Fractions were collected when MM 45289 started to elute from the column (6 minutes) and 2 x 1 minute fractions were collected, followed by 0.5 minute fractions. Fractions were assayed on a Waters analytical hplc column as described in the Detection Methods section.

Fractions 2 - 4 from both runs, containing MM 45289, free of MM 47756 were combined. The product was desalted by treatment with D-alanyl-D-alanine Sepharose affinity resin as described elsewhere and the affinity eluate evaporated *in vacuo* and freeze dried to yield 23mg of MM 45289 containing less than 1% MM 47756.

Example 5

Preparation of MM 45289 by preparative isoelectric focusing

Semi-purified extracts of MM 45289 may be further purified by preparative isoelectric focusing as described below.

200mg of partially purified MM 45289 was dissolved in 50ml of distilled water and 1% Bio-Lyte ampholyte 3/10 added [supplied by Bio-Rad Laboratories, 1414, Harbour Way South, Richmond. CA, USA]. The solution was loaded into the focusing chamber of a Bio-Rad Rotofor preparative isoelectric focusing cell. [Rotofor is a Trade Mark]. Fractionation was carried out at 12W constant power for 4 hours at 5°C. 20 fractions were harvested from the focusing chamber. The fractions were assayed by hplc. Fractions 15-19 (pH 8.6 - 9.4), containing MM 45289, were bulked. made up to 50ml with distilled water and re-loaded into the Rotofor focusing chamber without the addition of more ampholyte. Focusing was carried out at 12W, constant power for 4 hours at 5°C, and the collected fractions assayed by hplc. Fractions containing MM 45289 (Fractions 13 - 18, pH 8.9 - 9.1) were bulked, adjusted to pH 6.5 and treated with D-alanyl-D-alanine Sepharose affinity resin as described in previous examples, to remove the ampholytes. The affinity eluate was evaporated *in vacuo* and freeze dried to yield 68.0mg of substantially pure MM 45289.

Example 6

(a) Fermentation

200ml of seed culture obtained as described in Example 1 was used to inoculate 15 litres of fermentation medium in a 20 litre stainless steel fermenter. The fermentation medium was identical to the seed stage medium except that to control foaming 0.1% v/v polypropylene glycol P2000 was added in the

fermentation medium before sterilization. [P2000 was supplied by KWR Chemicals Ltd 133-35 Eleanor Cross Road, Waltham Cross, Herts.] The medium was steam sterilized in the fermenter for 60 minutes at 120°C. The fermentation was carried out for 93 hours at 28°C and 200 r.p.m. stirred by a three-vaned disc impeller of diameter 100 mm and supplied with 7.5L min sterile air. The harvested broth was clarified by centrifugation. The clarified broth contained MM47756 and MM45289.

b) Isolation of MM 47756

A 4 litre aliquot from the 12 litres of clarified broth obtained from the fermentation was loaded at 10ml/min onto an 'Amberlite' resin IRC-50 cation exchange column (Na+ form) (55 x 180cm) [The resin was supplied by Rohm and Haas UK Ltd, Lenning House, 2 Masons' Ave., Croydon UK].

The resin was washed with water (1L) and the antibiotics MM 47756 and MM 45289 eluted with 1M NaCl at a flow rate of 3ml/min. 400ml fractions were collected. The fractions were bioassayed by hole-in-plate using S.aureus V573. Antibacterially active fractions (1-5), containing a mixture of MM 7756 and MM 45289, were bulked and stirred with D-alanyl-D-alanine Sepharose affinity resin (30ml wet volume) for 1 hour.

The resin was filtered and washed with water (4 x 400ml) and then eluted with 0.1M ammonia containing 50% acetonitrile (2 x 200ml). The eluate was evaporated under reduced pressure to yield a cream solid (94mg).

The solid was taken up in 0.05M sodium phosphate buffer pH 7.0 (50ml) and loaded onto a CM Sephadex C25 cation exchange column (28 x 2.5cm) packed in 0.05M sodium phosphate buffer pH 7.0. ("Sephadex" is a Trade Mark.) (The resin was supplied by Pharmacia Ltd, Uppsala, Sweden). The column was washed with 0.05M sodium phosphate buffer pH 7.0 (400ml) and the glycopeptides then eluted using an exponential gradient of 0.05M sodium phosphate buffer pH 7.0 to pH 10.0, (250ml in mixing chamber) at a flow rate of 3ml/min. 10ml fractions were collected. Those fractions showing antibacterial activity were further monitored by hplc. Fractions 75 - 84 contained predominently MM 47756. Fractions 85 - 108 contained MM 45289. Fractions which contained predominantly MM 47756 were bulked. The pH of the bulked fractions was adjusted to 6.5 and the solution was stirred for 1hr with D-alanyl-D-alanine Sepharose affinity resin, (30ml wet volume). The resin was washed with water, and eluted with 0.1M ammonia containing 50% acetonitrile as previously described. The eluate fractions were bulked and evaporated to yield 23mg of MM 47756.

The solid was taken up in 0.05M sodium phosphate pH 7.0 and loaded onto a second CM Sephadex column (25 x 105cm). The column was washed and gradient eluted as described for the first CM Sephadex column, excepting the flow rate was 2ml/min and 5ml fractions were collected. Antibacterially active fractions were further monitored by hplc. Fractions containing MM 47756 (fractions 58 - 61) were bulked, adjusted to pH 6.5 and desalted with D-alanyl-D-alanine Sepharose affinity resin as previously described. The affinity resin eluate was evaporated to dryness and further purified by taking the solid up in water (10ml) and submitting it to a C18 Sep-pak cartridge [supplied by Waters Associates, 34 Maple Street, Milford, Mass., USA]. The cartridge was washed with water (2 x 10ml) and the glycopeptide was eluted with 50% methanol containing 0.1M ammonia (10ml). The eluate was evaporated to yield 4.5mg of MM 47756 substantially free of MM45289. Fast Atom Bombardment mass spectroscopy indicated a molecular weight of 1522±1.

The antibacterial activity of MM 47756 against a range of Gram positive organisms is shown in the following Table:

| Antibacterial activity of MM 47756 against a range of organisms, determined by the microtitre method (MIC µg/ml) | |
|---|---|
| Organism | MM 47756 |
| Bacillus subtilis ATCC 6633 | 0.25 |
| Coynebacterium xerosis NCTC 9755 | 1.0 |
| Sarcina lutea NCTC 8340 | 1.0 |
| Staphylococcus aureus | |
| 'Oxford' | 0.25 |
| 'Russell' | 2.0 |
| 'V573' MR* | 0.5 |
| S.saprophyticus 'FLI' | 1.0 |
| S.epidermidis | |
| 60137 | 1.0 |
| 54815 | 1.0 |
| Streptococcus pyrogenes CN10 | 0.12 |
| S.agalactiae 'Hester' | 0.5 |
| S.sanguis ATCC 10556 | 1.0 |
| S.pneumoniae Pu7 | 0.5 |
| S.faecalis 1 | 2.0 |

*Multi-resistant (Methicillin, tetracycline, erythromycin and gentamycin resistant)

Characterising data

Physical and spectral properties of MM 45289 after drying over $P_2O_5$:
FAB-MS (glycerol/thioglycerol/trifluoroacetic acid) $MH^+$ 1557±1
Molecular Weight: 1556
Molecular Formula: $C_{73}H_{89}N_{10}O_{26}Cl$
UV ($H_2O$) $\lambda_{max}$ 280 nm ($\epsilon$ 6215)
$[\alpha]_D^{20}$ -118° (c = 0.97% in $H_2O$)
IR (kBr) 1660, 1590, 1500, 1070 $cm^{-1}$
$^1H$ NMR in DMSO $d_6$ at 358°K. Tetramethylsilane as internal standard.
δH 8.45 (1H, broad s), 8.23 (1H, br d, J ca.5Hz), 7.95 (1H, br d, J ca.5Hz), 7.63 (1H, d, J 8.0Hz), 7.52 (1H, d, J 8.3Hz), 7.39 (1H, s), 7.33 (1H, br d), 7.23 (1H, d, J 8.3Hz), 7.12 (1H, s), 7.1 (1H, br d), 6.97 (1H, d, J 8.0Hz), 6.76 (1H, dd, J 8.4 and 2.0Hz), 6.68 (1H, d, J 8.4Hz), 6.45 (1H, d, J 2.0Hz), 6.33 (1H, d, J 2.0Hz), 5.68 (1H, br), 5.63 (1H, s), 5.50 (1H, d, J 7.4Hz), 5.43 (1H, s), 5.27 (2H, overlapping), 5.13 (1H, d, J ca.2Hz), 4.70 (2H, overlapping), 4.50 (1H, d, J 6Hz), 4.47 (1H, d, J 6.1), 4.33 (1H, br), 4.21 (1H, br), 4.13 (1H, dq, J 6.0 and 9.6Hz), 3.75 - 3.30 (7H, overlapping), 3.00 (1H, dd, J 7.8 and 5.8Hz) 2.88 (1H, d, J 9.6Hz), 2.86 (IH, d, J 9.6Hz), 2.58 (IH, dd, J 15.8 and 4.0Hz), 2.32 (3H, s), 2.17 (IH, dd, 15.8 and 6.8Hz), 1.90 (1H, br d, J ca.13Hz), 1.82 (1H, d, J 13.7Hz), 1.77 (1H, m), 1.60 (2H, overlapping, J 13.5 and 4.4Hz), 1.52 (1H, m), 1.42 (1H, m), 1.18 (3H, d, J 5.9Hz), 1.13 (6H, s), 1.09 (3H, d, J 6.0Hz), 0.92 (3H, d, J 6.6Hz), 0.89 (3H, d, J 6.6Hz) ppm.
Acid Hydrolysis (6MHCl/110° 18hrs/$N_2$ sealed tube) gives aspartic acid and N-methyl leucine.

Physical and spectral data on MM 47756:
FAB-Ms (thioglycerol/glycerol) $MH^+$ 1523±1
Molecular Weight: 1522
Molecular Formula: $C_{73}H_{90}N_{10}O_{25}$
$^1H$ NMR in DMSO $d_6$ at 353°K, Tetramethylsilane as internal standard. Inter alia 7.61 (2H, d, J ca.8.0Hz),

7.4 - 7.2 (3H, overlapping m), 7.13 (1H, dd, J 8.0 and 2.0Hz), 7.10 (1H, d, J 2.0 Hz), 7.07 (1H, dd, J 8.0 and 2.0Hz), 6.97 (1H, dd, J 8.0 and 2.0Hz), 6.76 (1H, dd, J 8.5 and 2.0Hz), 6.68 (1H, d, J 8.5Hz), 6.40 (1H, d, J 2.2Hz), 6.35 (1H, d, J 2.2Hz) ppm.

## Detection Methods

a) Fermentation samples and column fractions were monitored for antibiotic activity by bioassay on *Staphylococcus aureus* V573, using the conventional hole in plate method.

b) MM 45289 and MM 47756 have a characteristic UV maximum at 281nm. Purified samples can be assayed using direct measurement of this absorbance.

c) Preparations of MM 45289 and MM 47756 were assayed on a Waters high performance liquid chromatography column (3.9 x 300mm) containing µbondapak C18 reverse phase material. [Waters Associates, 34 Maple Street, Milford, Mass. USA.]. The glycopeptides were eluted from the column with 0.1M $NaH_2PO_4$ pH 6.0 containing 9% acetonitrile at a flow rate of 2ml/min. The eluant was monitored at 210 and 280nm by a Hewlet-Packard 1040A diode assay hplc monitor (Hewlet-Packard, Corvallis Division, 100 N.E. Circle Boulevard, Corvallis, Oregon, USA] or a Cecil model CE 2112 UV monitor [Cecil Instruments Ltd., Milton Industrial Estate, Milton, Cambridge, UK]. Under these conditions MM 45289 and MM 47756 had retention times of 4.2 and 5.8 minutes respectively (cf. vancomycin 11.2 minutes).

Using the same hplc system, but eluting with 0.1M sodium phosphate buffer pH 7.5 containing 15% acetonitrile and 0.005M sodium heptane sulphonate ion-pairing reagent, MM 45289 and MM 47756 had a retention time of 7.6 and 3.6 minutes respectively. With 0.1M sodium phosphate buffer pH 6.0 containing 12% acetonitrile and 0.005M sodium heptane sulphonate ion-pairing reagent, MM 45289 and MM 47756 had retention times of 11.8 minutes and 9.0 minutes (cf. vancomycin 7.0 minutes).

## Reference Example

### Preparation of affinity adsorbent

The N-hydroxysuccinimide ester of 6-aminohexanoic acid Sepharose 4B (60g) was placed on a glass scinter and washed with 1mM hydrochloric acid solution (2L) under suction. The wet cake was then added to a solution of D-alanyl-D-alanine (1.5g) in 0.1M sodium bicarbonate solution (60ml) and occasionally shaken over the next hour. The suspension was filtered under suction and the residue suspended in 0.1M tris (hydroxymethyl)aminomethane (TRIS) (100ml) for 1 hour and then refiltered through a glass sinter. The cake was washed successively with 0.1M sodium bicarbonate solution, 0.05M TRIS (containing 0.5M sodium chloride), 0.05M formate buffer at pH 4.0 (containing 0.5M sodium chloride) and finally distilled water. The affinity resin was then stored at 4°C in aqueous suspension.

## Claims

Claims for the following Contracting State: GR

1. The process for the production of a compound of formula (I):

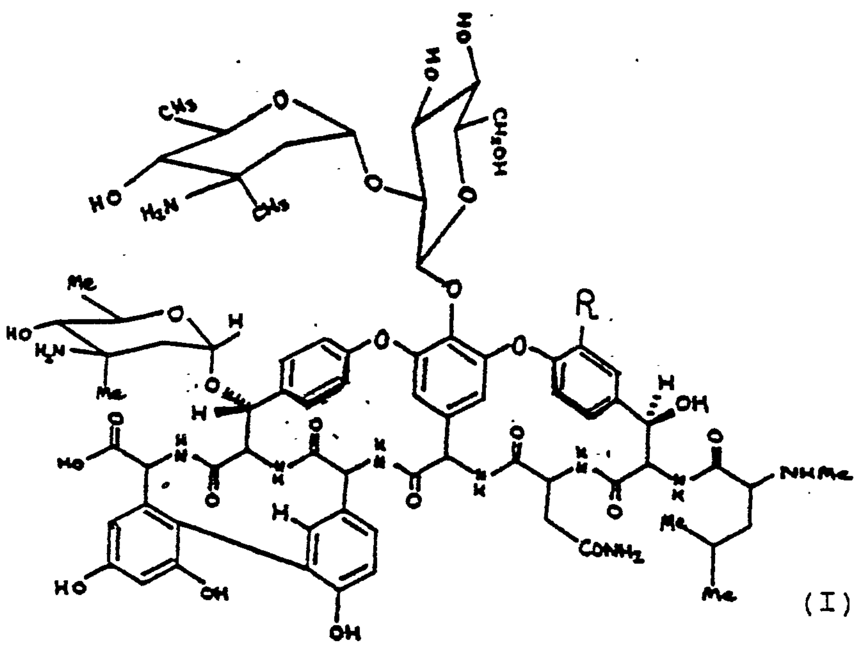

(I)

wherein R is hydrogen or chlorine, which process comprises cultivating Amycolatopsis orientalis NCIB 12531 or a mutant thereof and subsequently isolating the compound of formula (I) or a derivative thereof from the culture.

2. A process for the preparation of a compound of formula (I) as defined in claim 1, which process comprises separating the compound of formula (I) or a derivative thereof from a solution thereof in admixture with other antibacterially active substances and/or inactive substances by adsorption onto an affinity resin.

3. Amycolatopsis orientalis NCIB 12531 or a mutant thereof, in biologically pure form.

4. A mixture of compounds of formula (I) as defined in claim 1, in substantially pure form.

5. The compound of formula (I) as defined in claim 1 wherein R is chlorine, or a pharmaceutically acceptable derivative thereof.

6. A compound according to claim 5, in substantially pure form.

7. A compound according to claim 6, which is at least 85% pure w/w.

8. A pharmaceutical composition comprising a compound according to claim 5, 6 or 7 together with a pharmaceutically acceptable carrier or excipient.

9. A composition according to claim 8, formulated as powder, granules, lozenges, cream, or suppositories.

10. A method of treating bacterial infections in animals including humans, which comprises administering a compound according to claim 5, 6 or 7, or a composition according to claim 8 or 9, to a patient in need thereof.

11. A method according to claim 10, wherein the said compound or composition is administered topically.

12. Use of a compound according to claim 5, 6 or 7 in the manufacture of a medicament for use in the treatment of bacterial infections in animals including humans.

13. A compound according to claim 5, 6 or 7, for use in the treatment of bacterial infections in animals including humans.

14. A compound according to claim 5,6 or 7 for use in therapy.

Claims for the following Contracting State: ES

1. A process for the production of a compound of formula (I):

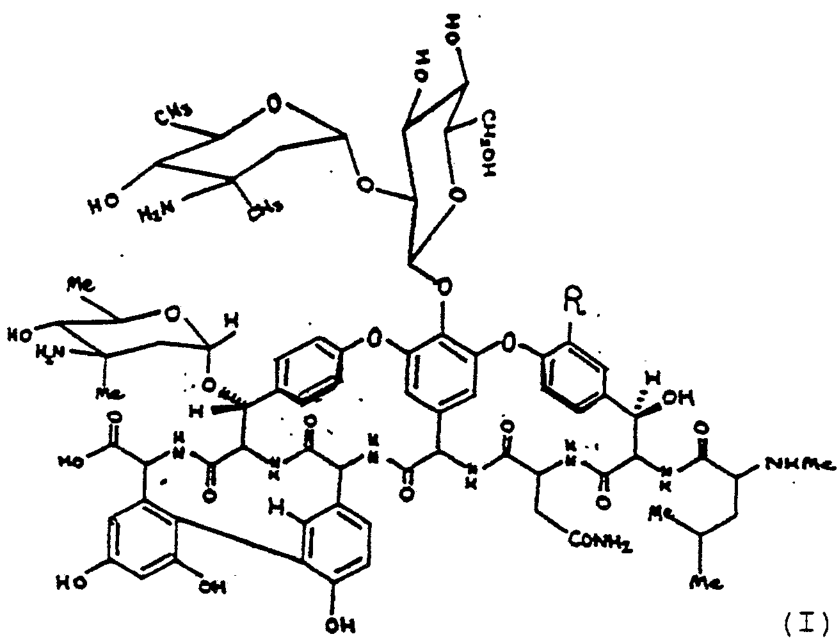

(I)

wherein R is hydrogen or chlorine, which process comprises (a) cultivating Amycolatopsis orientalis NCIB 12531 or a mutant thereof and subsequently isolating the compound of formula (I) or a derivative thereof from the culture, and or (b) separating the compound of formula (I) or a derivative thereof from a solution thereof in admixture with other antibacterially active substances and/or inactive substances by adsorption onto an affinity resin.

2. Amycolatopsis orientalis NCIB 12531 or a mutant thereof, in biologically pure form.

3. Use of the compound of formula (I) as defined in claim 1 wherein R is chlorine in the manufacture of a medicament for use in the treatment of bacterial infections in animals including humans.

4. Use according to claim 3, wherein the medicament is formulated as powder, granules, lozenges, cream, or suppositories.

5. Use according to claim 3 or 4, wherein the said medicament is administered topically.

6. Use according to claim 3, 4 or 5, wherein the said compound is substantially pure.

7. Use according to claim 6, wherein the said compound is at least 85% pure w/w.